# EUROPEAN PATENT APPLICATION

(11) **EP 4 390 797 A1**
(43) Date of publication of application: **26.06.2024**
(21) Application number: 22216044.2
(22) Date of filing: 22.12.2022
(51) Int. Cl.: G06Q 10/0631

(54) **SYSTEM AND METHOD TO ASSESS AND ENHANCE A PATIENT'S VIRTUAL CARE READINESS**

(71) Applicant: Koninklijke Philips N.V., 5656 AG Eindhoven (NL)
(72) Inventor: VAN BERKEL, Joep, Eindhoven (NL); VELTHOVEN (ep. DESSAUD), Nathalie, Eindhoven (NL); LACROIX, Joyca Petra Wilma, 5656AG Eindhoven (NL); DONGRE, Chaitanya, Eindhoven (NL)
(74) Representative: Philips Intellectual Property & Standards

(57) **Abstract**

A method of providing all or portions of a care program for a patient includes receiving data regarding the patient from a number of sources. At least some of the data is then used in determining a virtual readiness profile for the patient. A number of care elements for the patient are then determined based on the patient's virtual readiness profile and then provided to the patient and/or a caregiver of the patient.

## Description

### FIELD OF THE INVENTION:

The disclosed concept relates generally to systems and method for assessing and enhancing the virtual care readiness of a patient. More particularly, the present invention relates to systems and methods for providing all, or portions of, a care program for a patient.

### BACKGROUND OF THE INVENTION:

As generally recognized, delivery of healthcare is changing rapidly as virtual care has recently went quickly from a "nice to have" service offered by a healthcare provider to an essential element in healthcare due to COVID as well as innovations in healthcare. The success of virtual care programs depends on the ability to address many challenges, ranging from integration of virtual care in clinical practice, interoperability, platform integrations and continuous patient engagement and virtual care readiness of the patient. In scaling virtual care, providers must consider such challenges to enable provision of high quality virtual or hybrid care and figure out how such challenges interact with existing health inequities, in order to avoid exacerbating disparities. Looking from the patient virtual care accessibility perspective, patient engagement and virtual care readiness and their related challenges, a rapid move to virtual care for certain segments of the populations could worsen health inequities rather than reduce them.

A challenge on virtual care readiness and virtual care accessibility relies on the ability of a provider to identify upfront whether a given patient would fare well generally on a virtual care program of some type/form, and if so whether such program should be completely virtual or a hybrid (i.e., both in-person and virtual) care program and identify, quantify and measure patient barriers to virtual care success; from a clinical outcome perspective to make sure optimal outcomes are realized, from a patient satisfaction perspective and from a financial/resources perspective to make first time right decisions by preventing onboarding patients that eventually drop out and/or be transferred to hybrid care or non-virtual care, thus wasting virtual care resources.

Findings from interviews with health care professionals as part of a research focusing on understanding the experiences and impact of a virtual care program have shown that healthcare professionals have no systematic approach to predict a patient's willingness, capabilities and likelihood of successful engagement in virtual care programs. Instead, such findings showed that healthcare professionals make their own personal predictions based on how they perceive a patient's skills, motivation and likelihood of succeeding. User data as well as healthcare provider (HCP) interview results have shown that many patients do not succeed or are not willing to start their participation in virtual care and also that a substantial number of patients stop their participation after some time.

### SUMMARY OF THE INVENTION:

Embodiments of the present invention improve from existing solutions by providing, as a first aspect, a method of providing all or portions of a care program for a patient. The method comprises: receiving data regarding the patient from a number of sources; determining a virtual readiness profile for the patient based on at least some of the data; determining a number of care elements for the patient based on the patient's virtual readiness profile; and providing the number of care elements to the patient and/or a caregiver of the patient.

The number of care elements may comprise a plurality of care elements. The plurality of care elements may comprise a care program. The plurality of care elements may comprise a number of virtual care elements. The plurality of care elements may comprise a number of non-virtual care elements.

Providing the number of care elements to the patient may comprise carrying out at least some of the care elements with the patient.

Receiving data regarding the patient from the number of sources may comprise receiving the data from a plurality of sources. Receiving the data regarding the patient from the plurality of sources may comprise receiving the data from the patient and a number of caregivers of the patient.

Determining the virtual readiness profile for the patient based on at least some of the data may comprise: providing the data to a trained neural network; and receiving the virtual readiness profile from the trained neural network.

The patient's virtual readiness profile may comprise a virtual readiness score.

As another aspect, a system for use in assessing virtual care readiness of a patient and providing a number of care elements for a care program for the patient is provided. The system comprises: a data ingestion module; a data analysis module in communication with the data ingestion module; and a care decision module in communication with the data analysis module, wherein: the data ingestion module is structured to receive data pertaining to the patient from various sources and communicate the data, in-whole or in-part, to data the analysis module; the analysis module is structured to receive and analyze the data communicated by the data ingestion module and create a virtual care readiness profile of the patient; and the care decision module is structured to receive the virtual care readiness profile from the data analysis module and determine therefrom the number of care elements for the patient and providing the number of care elements for carrying out by the patient.

The data analysis module may include, in-whole or in-part, a trained neural network that has been trained to create a virtual care readiness profile of the patient from the data pertaining to the patient.

The virtual care readiness profile may be a single dimensional representation of the patient.

The virtual care readiness profile may be a multi-dimensional representation of the patient.

The virtual care readiness profile may comprise a virtual readiness score.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various Figs. It is to be expressly understood, however, that the drawings are for the purpose of illustration and description only and are not intended as a definition of the limits of the invention.

### BRIEF DESCRIPTION OF THE DRAWINGS:

A full understanding of the invention can be gained from the following description of the preferred embodiments when read in conjunction with the accompanying drawings in which:
FIG. 1 is a schematic representation of a system in accordance with an example embodiment of the present invention; and
FIG. 2 is a flow chart showing general steps of a method in accordance with an example embodiment of the present invention.

### DETAILED DESCRIPTION OF THE INVENTION:

Embodiments of the present invention generally revolve around defining risk of patients dropping out of virtual care programs due to lack of patients' adherence or patients' unreadiness towards virtual care programs and using such risk(s) to define care decision suggestions on what (combination of) virtual care elements, if any, would be optimal for a particular patient or a group of patients in order to seamlessly onboard such patient(s) to the right level of virtual care (or to determine that virtual care is not appropriate). Accordingly, embodiments of the present invention provide systematic means to assess virtual care readiness (or lack thereof) of a patient so as to increase the likelihood of successful participation in virtual care. Moreover, embodiments of the present invention may additionally provide recommendations of the virtual care program elements that should be offered to a patient so as to optimize the likelihood of successful participation by the patient in a program.

Upfront assessment and estimation of a patient's virtual care readiness and estimation of success by means of measuring both obtrusively and non-obtrusively patient features allows providers to conduct evidence-based selection and subsequent enrollment of the population for their virtual care (VC) programs.

Optimal patient selection for virtual or hybrid care programs such as described herein greatly reduce dropout and patient deterioration due to non-compliance, prevention or reduction of waste and resources and curb costs as patients (who are deemed ready for at least some extent of virtual care) are enrolled into the programs that best fit their particular needs and level of virtual care readiness, thus reducing patient drop-outs and or care transfers, reducing patient frustration and worsening willingness to self-manage, and increasing patient and provider satisfaction for making first time right (virtual) care onboarding decisions.

Translating the approach to a patient population implementation will also allow providers & payers to agree on payment models per segment as a stable pool of adherent managed population segment (although individuals might move in and out of the pre-assigned segment) as opposed to individual patients with varying risks of drop-out, as well as measure the virtual care readiness of different patient populations benchmarked to one another across providers or medical conditions.

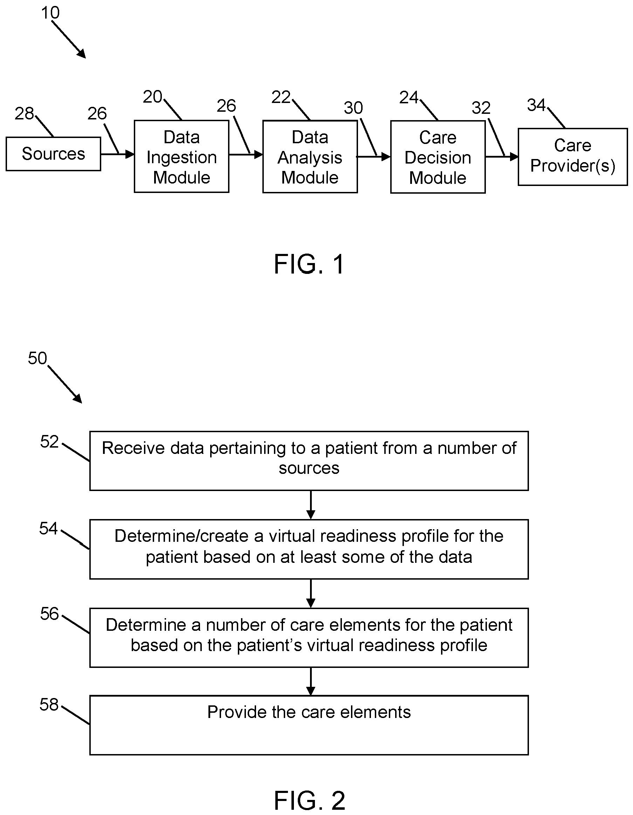

A system 10 for use in assessing virtual care readiness of a number of patients and recommending a number of care approaches for one or more of the number of patients in accordance with one example embodiment of the present invention is depicted generally in FIG. 1. As used herein the term "number" shall mean one or an integer greater than one (i.e., a plurality). System 10 includes a data ingestion module 20, a data analysis module 22, and a care decision module 24. Each of such modules 20, 22, 24 may comprise an independent processing arrangement or one or more of such modules may share a common processing arrangement depending on the needs of a particular embodiment. As used herein, the term "processing arrangement" is used to refer to any suitable electronic arrangement(s) for carrying out the functionality or functionalities indicated. In some example embodiments of the present invention, a processing arrangement comprises a microprocessor (µP) that interfaces with a memory module which can be any one or more of a variety of types of internal and/or external storage media such as, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory. As discussed further below, in an example embodiment of the present invention data analysis module 22 utilizes a processing arrangement comprising, in-whole, or in-part, a trainable/trained neural network of commonly known architecture. In another example embodiment, data analysis module 22 and care decision module 24 are generally one in the same module utilizing a common processing arrangement comprising, in-whole, or in-part, a trainable/trained neural network of commonly known architecture.

Data ingestion module 20 is structured to receive data 26 pertaining to a patient from various sources 28 and communicate data 26, in-whole or in-part, to data analysis module 22. Generally speaking, sources 28 include, for example, without limitation, one or more of the number of patients themselves, caregivers of patients and/or the medical records thereof or other information related thereto, and/or public databases and other public sources. Some more particular examples of sources 28 and/or data 26 pertaining to a patient include, but are not limited to:
Claims based data; insight into hospital (re-)admissions, interventions, clinical diagnosis;
History on behavior; adherence to agendas/appointment/no-shows for example from claims-based data or Electronic Medical Records (EMR) system on hospital appointments and effectiveness of previous home-based interventions (ECG holter, medication adherence etc.);
Informal Care Giver (ICG) care circle to identify what the patients care giver circle looks like and who is available to support;
Assess tech-savvy and willingness/engagement through mobile app/website navigation skills, ability to make online payments/purchasing, medical portal use, GP interactions (visit, phone, or online appointment scheduling);
Response of patient to presenting patient with intervention and timelines/journeys of virtual care programs, standard or routine care programs and hybrid tailored programs and highlight differences and expectations and burden to, and of, the patient;
Questionnaires to evaluate patient response and attitude towards self-management, health literacy, willingness to take accountability of their own care and the various forms of care delivery;
Actual tests to evaluate user virtual care readiness and willingness. BP measurement actual measurements and related measurement success rates and adherence. Expectation is that patient response will vary with time due to patient learning and reduced benefit of measurements but can still be used to profile patient and their self-management willingness and competences; and
Actual and historical measurement results that show whether and for how long the patient's measurements are well under control (often referred to as 'stable' patients by HCP) or not. For stable patients, the relevance of continuous and regular measurements is less critical than for unstable patients, thereby potentially impacting their optimal level of virtual care program engagement or the specific optimal set-up of the virtual care program.

Data analysis module 22 is structured to receive and analyze data 26 communicated by data ingestion module 20. As previously discussed, in an example embodiment of the present invention data analysis module 22 includes, in-whole or in-part, a trainable/trained neural network of commonly known architecture that has been trained to determine/create a virtual care readiness profile 30 of a patient from data 26 received from data ingestion module 20. As used herein, a "virtual care readiness profile" is a single or multi-dimensional representation of a given patient that can be used to compare one or more characteristics of a given patient with profiles of other patients so that the given patient and patients may be grouped, ranked or otherwise sorted. In one example embodiment of the present invention, a patient's virtual readiness profile 30 comprises a virtual readiness score. In such example, a range of scores, e.g. 1-100, may be possible, with a higher score indicating a patient is more prepared/ready for virtual care and a lower score indicating a patient is less/not prepared/ready (or vice versa). In another example embodiment of the present invention, a patient's virtual care readiness profile 30 includes a set of characteristics that describe the patient (e.g., data from the data ingestion module), and which describe the group the patient would be assigned to based on similarity to other patients and their features, including the above features and their observed success and way of participation in the virtual care program.

Care decision module 24 receives a patient's virtual care readiness profile 30 from data analysis module 22 and interprets the data therein to determine a number, typically a plurality, of appropriate care elements 32 for the patient. As used herein, a "care element" is an individual intervention with a patient, e.g., without limitation, an interaction with a doctor/physician assistant/nurse/therapist/etc., vital measurement (e.g., blood pressure/SpO2/etc., etc. Such care generally are the building blocks of a care program. In other words, a plurality of such individual care elements form a care plan for a patient. A care element may be of a virtual or non-virtual nature and a care program may include a mix of such virtual and non-virtual elements or merely only one or the other. For example, a vital measurement (e.g., blood pressure) might be carried out on a patient by a nurse in an office or alternatively by a patient on themself at home. Similarly, an interaction with a doctor might be carried out in-person or via a suitable teleconferencing arrangement (e.g., Zoom, TEAMS, FaceTime, etc.).

Care decision module 24 provides as output the number of care elements 32 for the particular patient to a care provider 34 (or providers, or other suitable recipient) for subsequently providing/carrying out with the patient. Depending on the amount of data 26 pertaining to a patient provided to data ingestion module, as well as the amount of data from other patient's available for reference and/or training of system 10, the quantity/level of complexity/detail of the number of care elements 32 provided by care decision module can greatly vary. For example, the number of care elements 32 can be of a very simple low detail result, e.g., merely providing the type of care program the patient should be provided, e.g., a solely virtual care program, a partially virtual care program (i.e., a hybrid care program), etc. In contrast, in an example embodiment wherein a larger amount of data is available for both the patient as well as data regarding outcomes from other patients, patients' risk of dropping out or risk of losing a patient due to lack of adherence/unwillingness or virtual care readiness to a program can be considered by system 10 in producing a multiple care elements and thus a care program. Based on virtual care readiness for selected tasks (along the dimensions as mentioned in the discussion of data ingestion module 20; ability or willingness to take measurements at home, commitment to an app, tech savviness, device ownership) care decision module 24 would define/provide suggestions on what (combination of) care elements (e.g., details of particular virtual care, details of other particular care, etc.) would be optimal for a particular patient in order to seamlessly onboard them to the right level of care including elements to train patients on the way to increase virtual care readiness.

A flow chart showing general steps of a method 50 in accordance with an example embodiment of the present invention such as may be carried out by system 10 is shown in FIG. 2. Method 50 generally begins at 52 wherein data pertaining to a patient is received from a number of sources (e.g., without limitation, such as previously described herein). Next, as shown at 54, a virtual readiness profile for the patient is determined/created based on at least some (the more the better) of the data received at 52. The patient's virtual readiness profile is then utilized to determine a number of care elements 32 for the patient, such as shown at 56. As shown at 58, the number of care elements 32 from 56 is then provided to a care provider (or providers, or other suitable recipient) for subsequently providing/carrying out with the patient (e.g., by one or more caregivers via suitable arrangement(s)).

It is contemplated that aspects of the disclosed concept can be embodied as computer readable codes on a tangible computer readable recording medium. The computer readable recording medium is any data storage device that can store data which can be thereafter read by a computer system. Examples of the computer readable recording medium include, for example, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

While specific embodiments of the invention have been described in detail, it will be appreciated by those skilled in the art that various modifications and alternatives to those details could be developed in light of the overall teachings of the disclosure. Accordingly, the particular arrangements disclosed are meant to be illustrative only and not limiting as to the scope of disclosed concept which is to be given the full breadth of the claims appended and any and all equivalents thereof.

## Claims

1. A method of providing all or portions of a care program for a patient, the method comprising:
receiving data regarding the patient from a number of sources;
determining a virtual readiness profile for the patient based on at least some of the data;
determining a number of care elements for the patient based on the patient's virtual readiness profile; and
providing the number of care elements to the patient and/or a caregiver of the patient.

2. The method of claim 1, wherein the number of care elements comprises a plurality of care elements.

3. The method of claim 2, wherein the plurality of care elements comprises a care program.

4. The method of claim 2, wherein the plurality of care elements comprises a number of virtual care elements.

5. The method of claim 4, wherein the plurality of care elements comprises a number of non-virtual care elements.

6. The method of claim 1, wherein providing the number of care elements to the patient comprises carrying out at least some of the care elements with the patient.

7. The method of claim 1, wherein receiving the data regarding the patient from the number of sources comprises receiving the data from a plurality of sources.

8. The method of claim 7, wherein receiving the data regarding the patient from the plurality of sources comprises receiving the data from the patient and a number of caregivers of the patient.

9. The method of claim 1, wherein determining a virtual readiness profile for the patient based on at least some of the data comprises:
providing the data to a trained neural network; and
receiving the virtual readiness profile from the trained neural network.

10. The method of claim 1, wherein the patient's virtual readiness profile comprises a virtual readiness score.

11. A system (10) for use in assessing virtual care readiness of a patient and providing a number of care elements for a care program for the patient, the system comprising:
a data ingestion module (20);
a data analysis module (22) in communication with the data ingestion module; and
a care decision module (24) in communication with the data analysis module,
wherein:
the data ingestion module is structured to receive data (26) pertaining to the patient from various sources (28) and communicate the data, in-whole or in-part, to data the analysis module;
the analysis module is structured to receive and analyze the data communicated by the data ingestion module and create a virtual care readiness profile of the patient; and
the care decision module is structured to receive the virtual care readiness profile from the data analysis module and determine therefrom the number of care elements for the patient and providing the number of care elements for carrying out by the patient.

12. The system of claim 11, wherein the data analysis module includes, in-whole or in-part, a trained neural network that has been trained to create a virtual care readiness profile of the patient from the data pertaining to the patient.

13. The system of claim 11, wherein the virtual care readiness profile is a single dimensional representation of the patient.

14. The system of claim 11, wherein the virtual care readiness profile is a multi-dimensional representation of the patient.

15. The system of claim 12, wherein the virtual care readiness profile comprises a virtual readiness score.
